# DEMANDE DE BREVET EUROPEEN

(11) **EP 1 671 678 A1**
(43) Date de publication de la demande: **21.06.2006**
(21) Numéro de dépôt: 05292564.1
(22) Date de dépôt: 02.12.2005
(51) Int. Cl.: A61Q 17/04, A61K 8/42, A61K 8/49

(54) **Composition solaire comprenant au moins un filtre uv organique insoluble et au moins une hydroxyalkyluree**

(30) Priorité: 20.12.2004 FR 0453077
(71) Demandeur: L'OREAL, S.A., 75008 Paris (FR)
(72) Inventeur: Candau, Didier, 91570 Bievres (FR)
(74) Mandataire: Miszputen, Laurent

(57) **Abrégé**

L'invention concerne une composition cosmétique ou dermatologique, à usage topique, comprenant dans un support cosmétiquement acceptable au moins un système filtrant les radiations UV, caractérisée par le fait qu'elle contient :
(a) au moins un filtre UV organique insoluble
(b) au moins une hydroxyalkylurée de formule (1) suivante 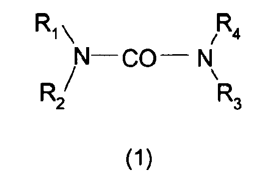
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que l'un de ses sels, solvats ou isomères.

L'invention concerne également l'utilisation d'une hydroxyalkylrée telle que définie ci-dessus dans une composition sous forme d'émulsion comprenant au moins un filtre UV hydrophile, dans le but d'améliorer le confort après application en particulier la douceur au toucher.

## Description

L'invention concerne une composition comprenant dans un support cosmétiquement acceptable au moins un système filtrant les radiations UV, caractérisée par le fait qu'elle contient :
(a) au moins un filtre UV organique insoluble
(b) au moins une hydroxyalkylurée particulière.

L'invention concerne également l'utilisation d'une hydroxyalkylurée de formule particulière dans une composition sous forme d'émulsion comprenant au moins un filtre UV hydrosoluble, pour améliorer le confort après application en particulier la douceur au toucher.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. II est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques lipophiles et/ou des filtres organiques classiques hydrophiles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

Les filtres UV les plus couramment utilisés sont organiques et solubles dans les huiles ou dans les milieux aqueux; ils possèdent généralement dans leur structure un groupe chromophore relié à un groupe solubilisant qui est généralement une chaîne grasse dans le cas des filtres UV liposolubles ou bien un groupe acide carboxylique ou sulfonique dans le cas des filtres UV hydrosolubles.

On connaît dans l'art antérieur des filtres UV organiques insolubles micronisés de taille moyenne de particule allant de 10 nm à 2 µm qui présentent l'avantage d'être plus efficaces que leurs homologues solubles comportant le même groupe chromophore à un taux équivalent. Ce type de filtres UV est notamment décrit dans les demandes de brevet EP746 305 et EP8405395. Cependant, certaines formulations solaires contenant ce type de filtre ont tendance après application à rendre la peau rêche.

Une autre difficulté réside dans le fait que les émulsions antisolaires à base de filtres UV insolubles, après application sur la peau, conduisent à une distribution irrégulière, non homogène voire grossière des filtres UV insolubles sur la peau, ce qui peut nuire à la qualité de l'effet de protection global recherché. Cette mauvaise répartition que l'on constate à la surface de la peau est souvent liée au fait qu'il existe, au niveau de l'émulsion un manque substantiel d'homogénéité après application.

Or, à la suite d'importantes recherches menées dans le domaine de la photoprotection évoqué ci-dessus, la Demanderesse a maintenant découvert, de façon inattendue et surprenante, qu'en ajoutant, dans ue composition solaire contenant au moins un filtre UV insoluble organique, une hydroxyalkylurée de formule (1) que l'on détaillera ci-après, il était possible d'améliorer sensiblement les propriétés cosmétiques comme le glissant, la douceur au toucher après application. Les compositions antisolaires contenant une telle association présentent en outre une bonne rémanence à l'eau, à la transpiration et aux lavages ainsi qu'une bonne persistance dans le temps.

Cette découverte est à la base de la présente invention.

Ainsi, conformément à l'un des objets de la présente invention, il est maintenant proposé une composition comprenant dans un support cosmétiquement acceptable au moins un système filtrant les radiations UV, caractérisée par le fait qu'elle contient :
(a) au moins un filtre UV organique insoluble et
(b) au moins une hydroxyalkylurée de formule (1) que l'on détaillera ci-après.

Un autre objet de l'invention est l'utilisation d'au moins une hydroxyalkylurée de formule (1) dans une composition comprenant dans un support cosmétiquement acceptable au moins un filtre UV organique insoluble, dans le but d'améliorer le confort après application en particulier la douceur au toucher.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Dans la suite de la présente description, on entend par « filtre UV organique insoluble », au sens de la présente invention, un composé organique filtrant les radiations UV ayant une solubilité dans l'eau inférieure à 0,1 % en poids et une solubilité inférieure à 1 % en poids dans la plupart des solvants organiques comme l'huile de paraffine, les benzoates d'alcools gras et les triglycérides d'acides gras.

D'autres caractéristiques, aspects et avantages de l'invention apparaîtront à la lecture de la description détaillée qui va suivre.

Les hydroxyalkyl urées conforment à l'invention sont choisies par celles répondant à la formule générale (1) : 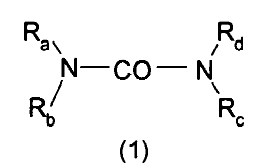 dans laquelle Rₐ, R_{b}, R_{c} et R_{d} représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux Rₐ-R_{d} représentant un groupe hydroxyalkyle, ainsi que leurs sels, solvats et isomères.

Dans la formule (1), parmi les groupes alkyle, on peut notamment citer les groupes méthyle, éthyle, n-propyle, isopropyle, n-butyle, iso-butyle et tert-butyle.

Les composés de formule (1) préférés sont ceux ne renfermant qu'un seul groupe hydroxyalkyle, c'est-à-dire ceux pour lesquels Rₐ est un groupe hydroxyalkyle et R_{b}, R_{c} et R_{d} représentent, indépendamment les uns des autres, un atome d'hydrogène ou un groupe alkyle en C₁-C₄. On préfère plus particulièrement les composés de formule (1) pour lesquels Rₐ est un groupe hydroxyalkyle et R_{b}, R_{c} et R_{d} représentent chacun un atome d'hydrogène.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle. Le groupe hydroxyéthyle est préféré.

Comme composés de formule (1) préférés, on peut citer la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée ; la N-méthyl-N-(1,3,4,5,6-pentahydroxy-2-hexyl)- urée; la N-méthyl-N'-(1-hydroxy-2-méthyl-2-propyl)- urée; la N-(1-hydroxy-2-méthyl-2-propyl)- urée; la N-(1,3-dihydroxy-2-propyl)- urée; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée; la N,N-Bis-(2-hydroxyéthyl)-N'-propyl- urée ; la N,N-Bis-(2-hydroxypropyl)-N'-(2-hydroxyéthyl)- urée ; la N-tert. Butyl-N'-(2-(hydroxyéthyl)-N'-(2-(hydroxypropyl)- urée; la N-(1,3-dihydroxy-2-propyl)-N'-(2-hydroxyéthyl)- urée; la N,N-Bis-(2-hydroxyéthyl)-N',N'-dimethyl- urée ; la N,N,N',N'-tetrakis-(2-hydroxyéthyl)- urée ; et la N',N'-Bis-(2-hydroxyéthyl)-N',N'-bis-(2-hydroxypropyl)- urée.

Un composé particulièrement préféré pour une utilisation dans la présente invention est la N-(2-hydroxyéthyl)-urée, ci-après désignée par "hydroxyéthyl urée".

Les hydroxyalkyl urées de formule (1) peuvent être préparées comme décrit dans la demande DE-27 03 185. Parmi celles-ci, l'hydroxyéthyl urée est en outre disponible dans le commerce, sous forme de mélange à 50% en poids dans l'eau, auprès de la société NATIONAL STARCH sous la dénomination commerciale Hydrovance®.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un
ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (1).

Les hydroxyalkylurées conformes à l'invention sont de préférence présentes dans les compositions conformes à l'invention à des teneurs de 0,01 à 50% en poids et plus préférentiellement de 0,1 à 20 % et encore plus préférentiellement de 0,1 à 10% en poids par rapport au poids total de la composition.

Les filtres UV organiques insolubles comportant au moins un, groupe absorbant le rayonnement UV peuvent être choisis notamment parmi les filtres UV organiques insolubles de type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.
Parmi les filtres UV insolubles de type oxalanilide, on peut citer ceux répondant à la formule : 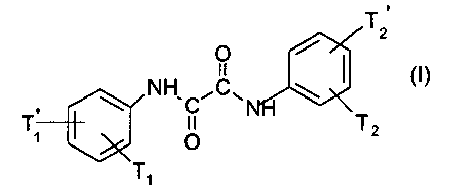 dans laquelle T₁, T₁', T₂ et T₂, représentent chacun indépendamment un radical alkyle en C1-C8 ou un radical alcoxy en C₁-C₈. Ces composés sont décrits dans la demande de brevet WO95/22959. A titre d'exemples, on peut citer les produits commerciaux TINUVIN® 315 et TINUVIN® 312 vendus par la Société CIBA-GEIGY correspondant respectivement aux formules :

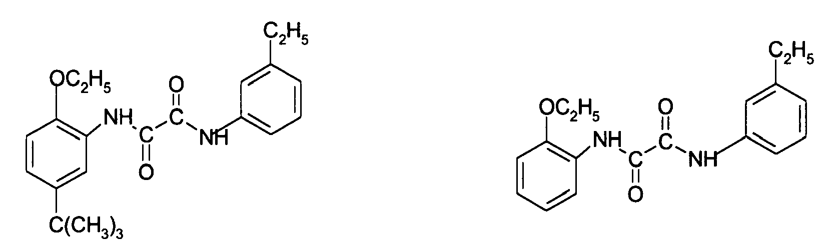

Les filtres insolubles de type triazine répondent à la formule générale suivante : 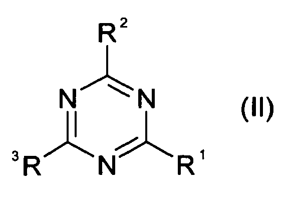 dans laquelle R¹, R² et R³ représentent chacun indépendamment un groupe phényle, phénoxy ou pyrrolo non substitués ou portant chacun indépendamment un, deux ou trois substituants choisis parmi -OH, alkyle en C1-18, alkoxy en C₁-C₁₈, carboxy(alkyle en C₁₋18), cycloalkyle en C₅-C₈, méthylbenzylidène-camphre, -(CH=CR')ₙ(CO)-OR⁴ où R' représente un atome d'hydrogène, un groupe cyano ou un groupe COOR⁴, avec R⁴ = alkyle en C₁-C₁₈ ou cinnamyle, et n vaut 0 ou 1.
Ces composés sont décrits dans WO 97/03643, GB 2286774, EP 743309, WO 98/22447, GB 2319523 et EP-A-0 790 243.

On citera plus particulièrement les composés suivants :
- la 2,4,6-tris(4'-amino benzalmalonate de diéthyle)-s-triazine,
- la 2,4,6-tris(4'-amino benzalmalonate de düsopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Parmi les filtres UV de type triazine utilisables pour la présente invention, on peut encore mentionner les dérivés insolubles de s-triazine portant des groupements benzotriazoles et/ou benzothiazoles tels que ceux décrits dans la demande WO98/25922.
Parmi ces composés, on peut citer plus particulièrement la 2,4,6-tris[(3'-benzotriazol-2-yl-2'-hydroxy-5'-methyl)-phenyl-amino]-s-triazine et la 2,4,6-tris[(3'-benzotriazol-2-yl-2'-hydroxy-5'-ter-octyl)-phényl-amino]-s-triazine.

Parmi les filtres UV organiques insolubles de type benzotriazole, on peut citer ceux de formule (III) ci-dessous, décrits par exemple dans la demande internationale WO95/22959 : 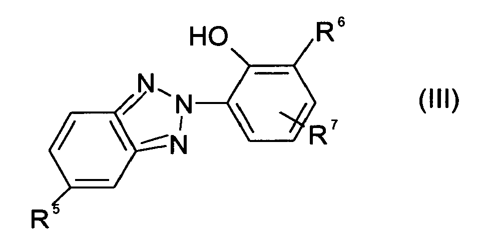 dans laquelle R⁵ désigne un atome d'hydrogène ou un radical alkyle en C₁-C₁₈, R⁶ et R⁷, identiques ou différents, désignent chacun indépendamment un radical alkyle en C₁-C₁₈ éventuellement substitué par un groupe phényle.

A titre d'exemples de composés de formule (III), on peut citer les produits commerciaux TINUVIN® 328 , 320, 234 et 350 de la Société CIBA-GEIGY correspondant respectivement aux formules suivantes :

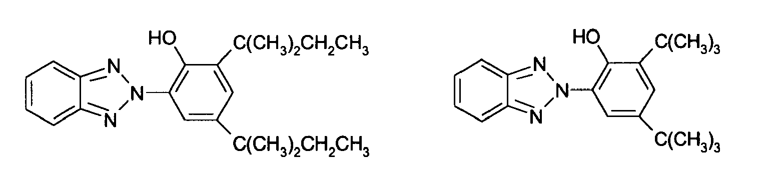

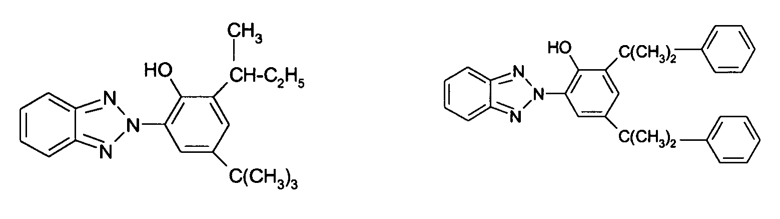

Parmi les filtres UV organiques insolubles de type benzotriazole, on peut encore citer les composés décrits dans les brevets US 5687521, US 5687521, US 5373037, US 5362881, et parmi eux en particulier le [2 ,4'-dihydroxy-3-(2H-benzotriazol-2-yl)-5-(1,1,3,3-tétraméthylbutyl)-2'-n-octoxy-5'-benzoyl]-diphénylméthane de formule : 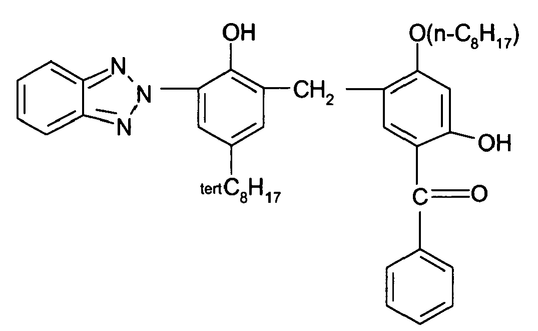 vendu sous le nom MIXXIM® PB30 par la société FAIRMOUNT CHEMICAL.

D'autres filtres UV organiques insolubles de type benzotriazole sont les dérivés de méthylène-bis-(hydroxyphénylbenzotriazole) de structure suivante : 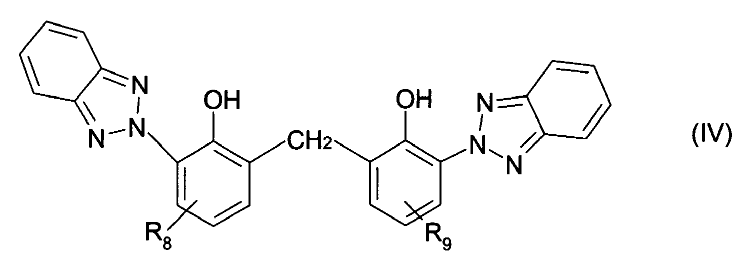 dans laquelle R⁸ et R⁹, identiques ou différents, représentent chacun un radical alkyle en C₁-C₁₈ pouvant être substitué par un ou plusieurs radicaux choisis parmi alkyle en C₁-C₄, cycloalkyle en C₅-C₁₂ ou aryle. Ces composés sont connus et sont décrits dans les demandes US 5237071, US 5166355, GB-A-2 303549, DE 19726184 et EP-A-893119.

Dans la formule (IV) définie ci-dessus, les groupes alkyle en C₁-C₁₈ peuvent être linéaires
ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle ; les groupes cycloalkyle en C₅-C₁₂ sont par exemple cyclopentyle, cyclohexyle, cyclooctyle ; les groupes aryle sont par exemple phényle, benzyle.

Parmi les composés de formule (IV), on préfère plus particulièrement ceux de structure suivante :

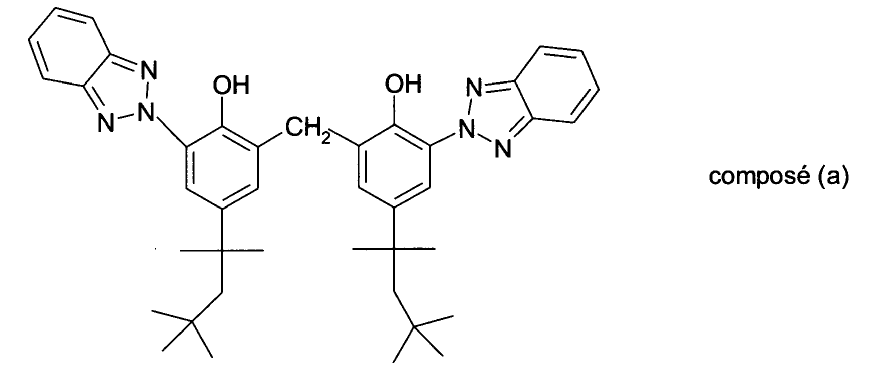

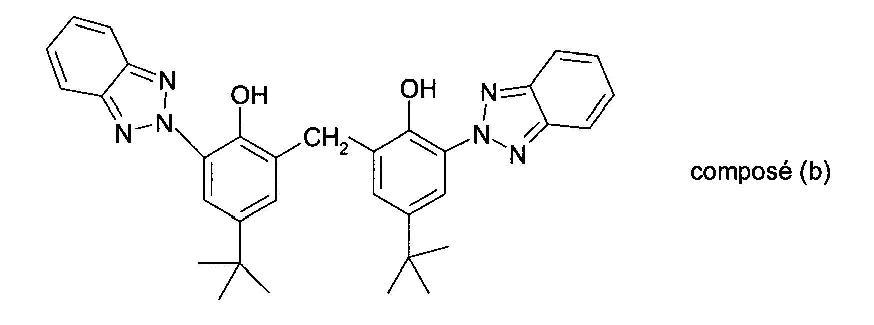

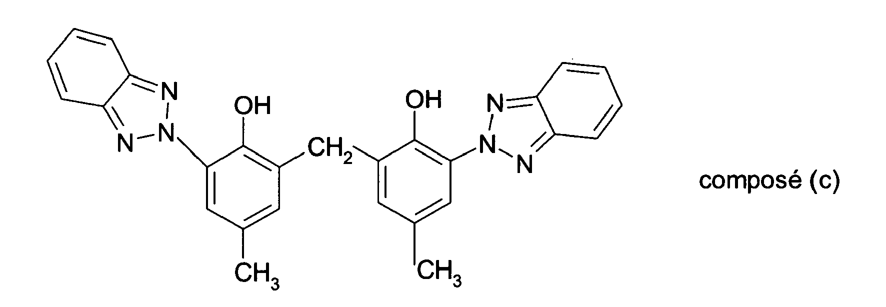

Le composé (a) de nomenclature 2,2'-méthylène-bis-[6-(2H-benzotriazol-2-yl)-4-(1,1,3,3-tetraméthylbutyl)phénol] est vendu sous forme solide le nom MIXXIM BB/100 par le société FAIRMOUNT CHEMICAL et sous forme micronisée sous le nom TINOSORB M par la société par CIBA SPECIALTY CHEMICALS.

Le composé (c) de nomenclature 2,2'-méthylène-bis-[6-(2H-benzotriazol-2-yl)-4-(methyl)phénol] est vendu sous forme solide sous le nom MIXXIM BB/200 par le société FAIRMOUNT CHEMICAL .

Parmi les filtres organiques insolubles du type amide vinylique, on peut citer par exemple les composés de formule (V) qui sont décrits dans la demande WO95/22959 :

T₃-(Y)r-C(=O)-C(T₄)=C(T₅)-N(T₆)(T₇) (V)

dans laquelle T₃ est un radical alkyle en C₁-C₁₈, de préférence en C₁-C₅, ou un groupe phényle éventuellement substitué par un, deux ou trois radicaux choisis parmi OH, alkyle en C₁-C₁₈, alcoxy en C₁-C₈ ou -C(=O)-OT₈ où T₈ représente un groupe alkyle en C₁-C₁₈ ; T₄, T₅, T₆ et T₇ représentent chacun indépendamment un radical alkyle en C₁-C₁₈, de préférence en C₁-C₅, ou un atome d'hydrogène ; Y représente un groupe -NH- ou un atome d'oxygène et r vaut 0 ou 1.

Parmi ces composés, on citera plus particulièrement :
la 4-octylamino-3-pentèn-2-one ;
le 3-octylamino-2-buténoate d'éthyle ;
la 3-octylamino-1-phényl-2-butèn-1-one
la 3-dodécylamino-1-phenyl-2-butèn-1-one.

Parmi les filtres organiques de type cinnamide, on peut citer les composés tels que ceux décrits dans la demande WO 95/22959 et répondant à la formule suivante : 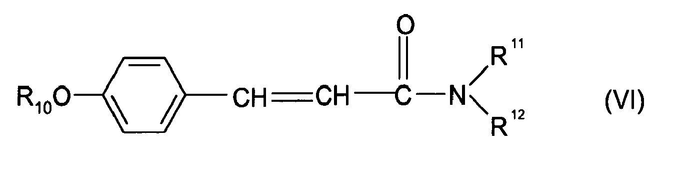 dans laquelle
R₁₀ représente un atome d'hydrogène ou un groupe alkyle en C₁-C₄ de préférence méthyle ou éthyle,
R¹¹ représente un atome d'hydrogène ou un groupe alkyle en C₁-C₄, de préférence méthyle ou éthyle,
R¹² représente un groupe -(CONH)ₛ-phényle où s vaut 0 ou 1 et le groupe phényle peut être substitué par un, deux ou trois groupes choisis parmi OH, alkyle en C₁-C₁₈, alcoxy en C₁-C₈ ou -C(=O)-OR¹³ où R₁₃ est un alkyle en C₁-C₁₈, et plus préférentiellement R¹² représente un groupe phényle, 4-méthoxyphényle ou phénylaminocarbonyle.

On peut également citer les dimères bis-[cinnamides 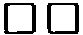 -disubstitués] , décrits par exemple dans le brevet US 5 888 481, de structure : 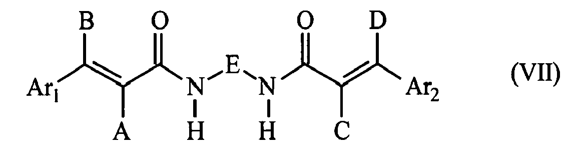 dans laquelle
Ar1 et Ar2, identiques ou différents, représentent chacun un radical phényle, un hétérocycle aromatique, un groupe à noyau phényle condensé ou un groupe à hétérocycle aromatique condensé, et peuvent porter un ou plusieurs substituants, identiques ou différents,
B et D, différents d'un atome d'hydrogène, représentent chacun indépendamment un radical organique,
A et C représentent chacun indépendamment un radical organique, et
E représente un radical organique divalent,
à l'exclusion des composés pour lesquels Ar1 et Ar2 représentent tous les deux un groupe phényle portant un substituant -OR où R représente un atome d'hydrogène ou un radical organique, A et C représentent tous les deux un groupe cyano, B et D représentent tous les deux un groupe alkyle ou alcényle en C₁-C₃₅, et E représente un radical organique divalent,
et en particulier le composé de structure :

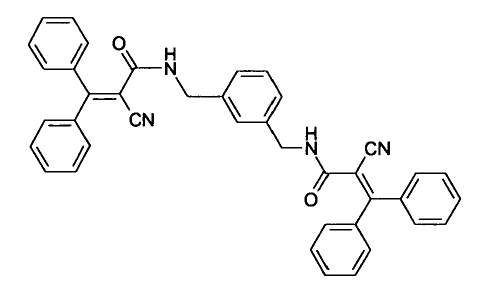

Parmi les filtres organiques insolubles du type benzazole, on peut citer ceux répondant à l'une des formules suivantes : 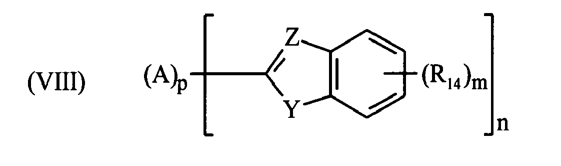 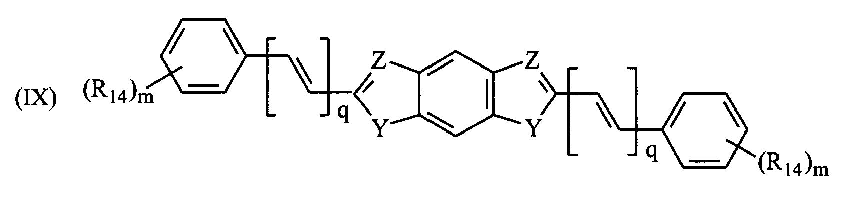 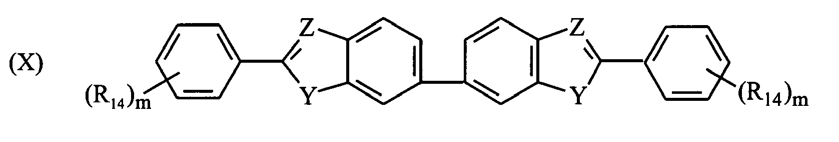 dans lesquelles
chacun des symboles Y représente indépendamment un atome d'oxygène ou de soufre ou un groupe NR₁₅,
chacun des symboles Z représente indépendamment un atome d'azote ou un groupe CH, chacun des symboles R₁₄ représente indépendamment un groupe OH, un atome d'halogène, un groupe alkyle en C₁-C₈, linéaire ou ramifié, contenant éventuellement un atome de silicium, ou un groupe alcoxy en C₁-C₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₁₅ représente indépendamment un atome d'hydrogène, un groupe benzyle ou alkyle en C₁-C₈, linéaire ou ramifié, contenant éventuellement un atome de silicium,
A représente un radical de valence n choisi parmi ceux de formules : 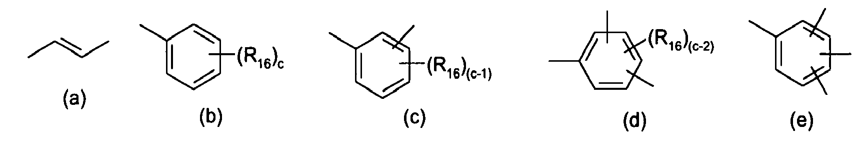 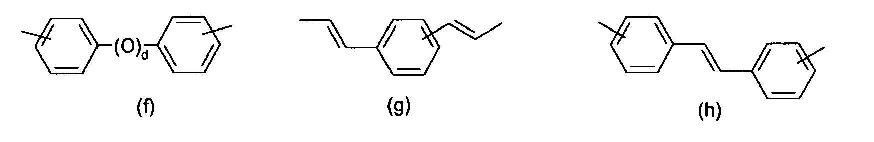 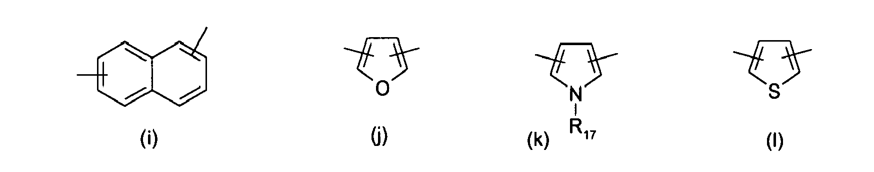 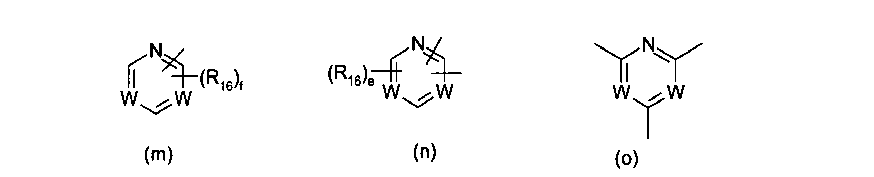 dans lesquelles chacun des symboles R₁₆ représente indépendamment un atome d'halogène ou un groupe alkyle ou alcoxy en C1-4, linéaire ou ramifié, ou hydroxy,
R₁₇ représente un atome d'hydrogène ou un groupe alkyle en C₁-C₄, linéaire ou ramifié, c = 0 - 4, d = 0 - 3, e = 0 ou 1 et f = 0 - 2.

Ces composés sont notamment décrits dans les brevets DE 676 103 et CH 350763, le brevet US 5501850, le brevet US 5961960, la demande de brevet EP 0669323, le brevet US 5518713, le brevet US 2463264, l'article du J. Am. Chem. Soc., 79, 5706 - 5708, 1957, l'article publié dans J. Am. Chem. Soc., 82, 609 - 611, 1960, la demande de brevet EP 0921126 et la demande de brevet EP 0712855.

A titre d'exemples de composés préférés de formule (VIII) de la famille des 2-arylbenzazoles, on peut mentionner le 2-benzoxazol-2-yl-4-méthylphénol, le 2-(1H-benzimidazol-2-yl)-4-méthoxyphénol ou le 2-benzothiazol-2-ylphénol, ces composés pouvant être préparés par exemple selon les procédés décrits dans le brevet CH 350 763.

A titre d'exemples de composés préférés de formule (VIII) de la famille des benzimidazolylbenzazoles, on citera le 2,2'-bis-benzimidazole, le 5,5',6,6'-tétraméthyl-2,2'-bis-benzimidazole, le 5,5'-diméthyl-2,2'-bis-benzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, ces composés pouvant être préparés selon les modes opératoires décrits dans les brevets US 5961960 et US 2463264.

A titre d'exemples de composés préférés de formule (VIII) de la famille des phénylène-benzazoles, on citera le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(N-triméthylsilylméthyl-2-benzimidazolyle), ces composés pouvant être préparés selon les modes opératoires décrits dans le brevet US 2 463 264 et dans les publications J. Am.Chem. Soc., 82, 609 (1960) et J. Am. Chem. Soc., 79, 5706 - 5708 (1957).

A titre d'exemples de composés préférés de formule (VIII) de la famille des benzofuranyl-benzoxazoles, on citera le 2-(2-benzofuranyl)-benzoxazole, le 2-(benzofuranyl)-5-méthylbenzoxazole et le 2-(3-méthyl-2-benzofuranyle)-benzoxazole, ces composés pouvant être préparés selon les modes opératoires décrits dans le brevet US 5518713.

Comme composés préférés de formule (IX), on peut citer par exemple le 2,6-diphényl-1,7-dihydro-benzo[1,2-d;4,5-d']-diimidazole correspondant à la formule 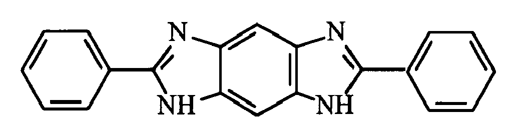 ou le 2,6-distyryl-1,7-dihydro-benzo[1,2-d ; 4,5-d']-diimidazole ou encore le 2,6-di(p-tert-butylstyryl)-1,7-dihydrobenzo[1,2-d ; 4,5-d']-diimidazole, qui peuvent être préparés selon les procédés décrits dans la demande EP 0 669 323.

Comme composé préféré de formule (X), on peut citer le 5,5'-bis-[(phényl-2)-benzimidazole] de formule : 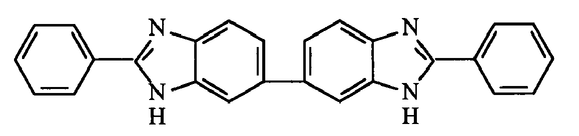 dont la préparation est décrite dans J. Chim. Phys., 64, 1602 (1967).

Parmi ces composés organiques insolubles filtrant le rayonnement UV, on préfère tout particulièrement le 2-(1H-benzimidazol-2-yl)benzoxazole, le 6-méthoxy-2,2'-bis-benzimidazole, le 2-(1H-benzimidazol-2-yl)-benzothiazole, le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(2-benzimidazolyle) et le 1,4-phénylène-bis-(N-triméthylsilylméthyl-2-benzimidazolyle).

Une autre famille de filtres insolubles est celle des arylvinylène-cétones choisies parmi celles correspondant à l'une des formules (XI) et (XII) suivantes : 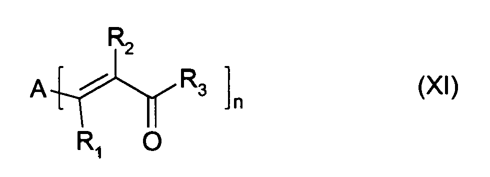 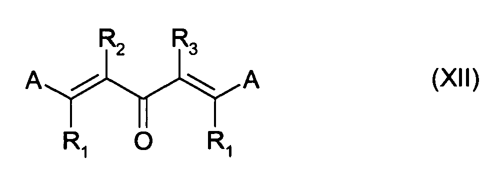 dans lesquelles :
n = 1 ou 2,
A, dans la formule (XI) lorsque n = 1 ou dans la formule (XII), est un radical aryle choisi parmi les formules (a) à (d) suivantes, ou dans la formule (XI) lorsque n=2, est un radical choisi parmi les formules (e) à (h) suivantes : 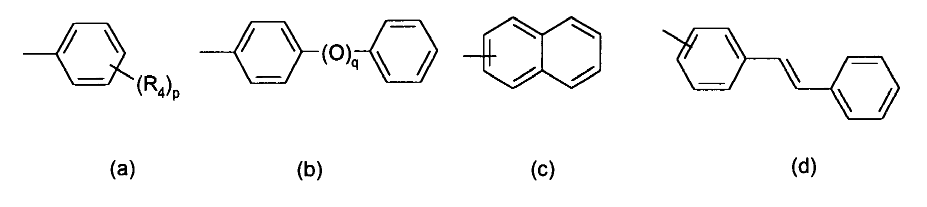 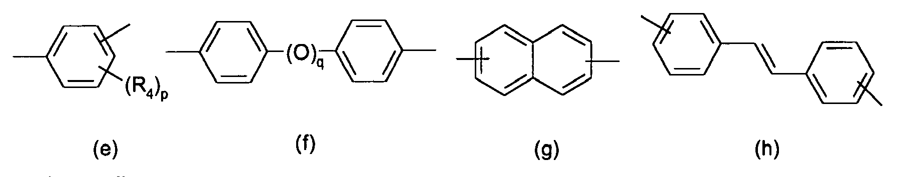 dans lesquelles :
chacun des symboles R₄ représente indépendamment un groupe OH, un atome d'halogène, un groupe alkyle en C₁-C₆, linéaire ou ramifié et contenant éventuellement un atome de silicium, un groupe alcoxy en C₁-C₆, linéaire ou ramifié et contenant éventuellement un atome de silicium, un groupe alcoxycarbonyle en C₁-C₅, linéaire ou ramifié, ou un groupe alkylsulfonamide en C₁-C₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁-C₆, linéaire ou ramifié et contenant éventuellement un atome de silicium, un groupe cyano, un groupe alkylsulfonyle en C₁-C₆, un groupe phénylsulfonyle,
R₃ représente un groupe alkyle en C₁-C₆, linéaire ou ramifié et contenant éventuellement un atome de silicium ou un groupe phényle pouvant former un bicycle et éventuellement substitué par un ou deux radicaux R₄,

ou R₂ et R₃ forment ensemble un reste hydrocarboné en C₂-C₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium ou une fonction aminoacide ; à condition que lorsque n=1, R₂ et R₃ ne forment pas un noyau camphre.

A titre d'exemples de composés de formule (XI) dans laquelle n=1, insolubles, filtrant le rayonnement UV, on peut mentionner les familles suivantes : Styryl Cétone (Kao JP 04 134 042) telle que la 1-(3,4-diméthoxy-phényl)-4,4-diméthyl-pent-1-èn-3-one : 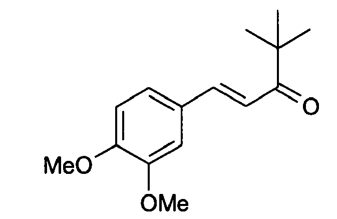 Benzylidène Cinéole (E. Mariani et al, 16th IFSCC Congress, New York (1990)) tel la 1,3,3-triméthyl-5-(4-méthoxy-benzylidène)-2-oxa-bicyclo[2.2.2]octan-6-one : 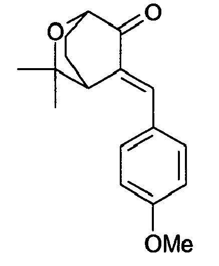 Benzylidène Chromanone (Kao JP 04 134 043) telle que la 3-(4-méthoxy-benzylidène)-2,3,4a,8a-tétrahydro-chromèn-4-one : 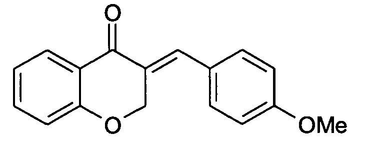 Benzylidène Thiochromanone (Kao JP 04 134 043) telle que la 3-(4-méthoxy-benzylidène)-2,3,4a,8a-tétrahydro-chromèn-4-thione : 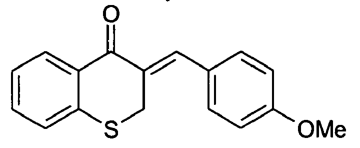 Benzylidène Quinuclidinone (Merck EP 0 576 974) telle que la 4-méthoxy benzylidène-1-aza-bicyclo[2.2.2]octan-3-one : 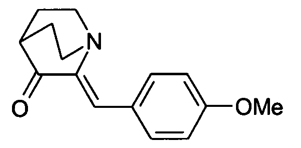 Benzylidène Cycloalcanone (Henkel FR 2 395 023) telle que les 2-(4-méthoxy-benzylidène)-cyclopentanone et 2-(4-méthoxy-benzyl-idène)-cyclohexanone : 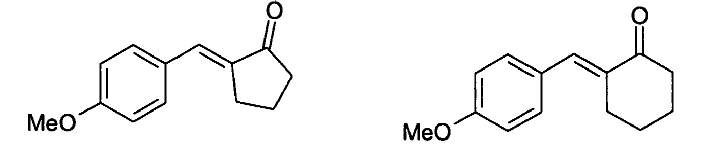 Benzylidène Hydantoïne (Ajinomoto JP 01 158 090) telle que la 5-(3,4-diméthoxy-benzylidène)-imidazolidine-2,4-dione : 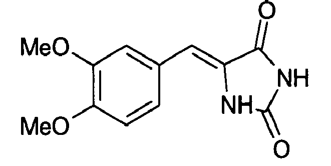 Benzylidène Indanone (Kao JP 04 134 043) telle que la 2-(4-méthoxy-benzylidène)-indan-1-one : 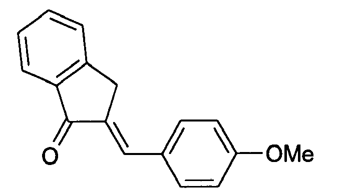 Benzylidène Tétralone (Kao JP 04 134 043) telle que la 2-(4-méthoxy-benzylidène)-3,4-dihydro-2H-naphthalen-1-one : 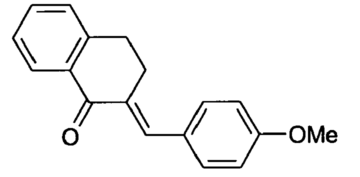 Benzylidène Furanone (L'Oréal EP 0 390 683) telle que la 4-(4-méthoxy-benzylidène)-2,2,5,5-tétraméthyl-dihydro-furan-3-one : 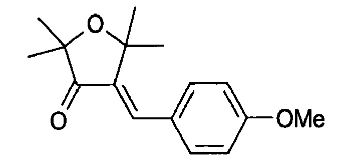 Benzylidène Benzofuranone (Kao JP 04 134 041) telle que la 2-benzylidène-benzofuran-3-one : 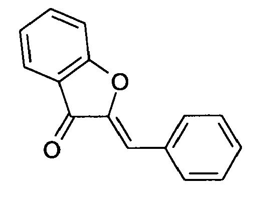 Benzylidène Indanedione telle que la 2-(3,5-di-tert-butyl-4-hydroxy-benzylidène)-indan-1,3-dione : 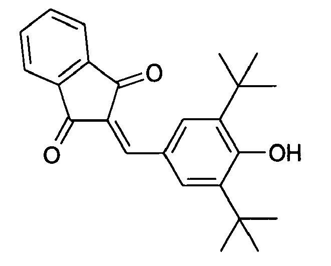 Benzylidène Benzothiofuranone (Kao JP 04,134,043) telle que la 2-benzylidène-benzo[b]thiophen-3-one : 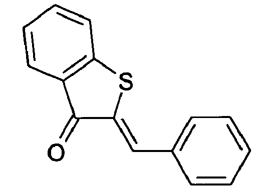 Benzylidène Barbiturique tel que la 5-(4-méthoxy-benzylidène)-1,3-diméthyl-pyrimidine-2,4,6-trione : 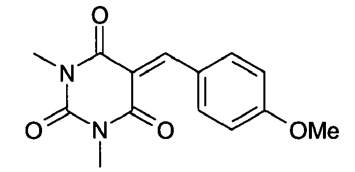 Benzylidène Pyrazolone telle que la 4-(4-méthoxy-benzylidène)-5-méthyl-2-phényl-2,4-dihydro-pyrazol-3-one : 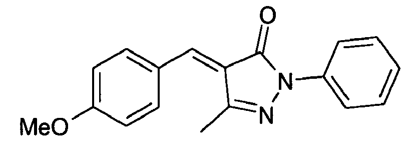 Benzylidène Imidazolone telle que la 5-(4-méthoxy-benzylidène)-2-phényl-3,5-dihydro-imidazol-4-one :

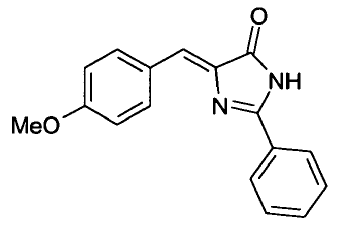

Chalcone telle que la 1-(2-hydroxy-4-méthoxy-phényl)-3-phényl-propènone : 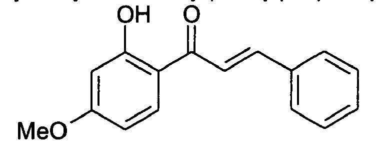 Benzylidène One (forme tautomère filtrante des dibenzoylméthanes ; L'Oréal FR 2 506 156) telle que la 3-hydroxy-1-(2-hydroxy-4-méthoxy-phényl)-3-phényl-propènone :

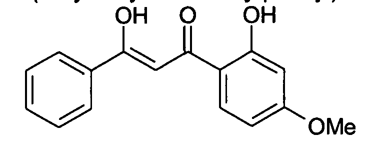

A titre d'exemples de composés de formule (XI) dans laquelle n=2 insolubles, filtrant le rayonnement UV, on peut mentionner les familles suivantes :
Phénylène bis méthylidène-nor-camphre (Merck EP 0 693 471) tel que la 1,4-phénylène-bis-{3-méthylidène-bicyclo[2.2.1]heptan-2-one} : 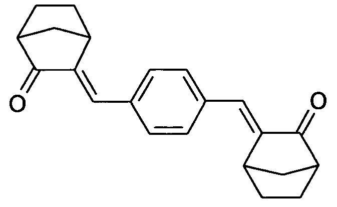 Phénylène bis méthylidène camphre (L'Oréal FR 2 528 420) telle que la 1,4-phénylène-bis-{3-méthylidène-1,7,7-triméthyl-bicyclo [2.2.1]heptan-2-one} : 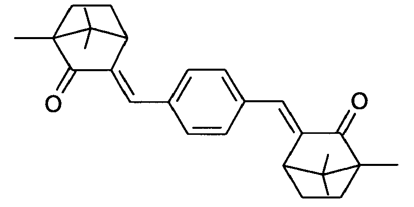 ou la 1,3-phénylène-bis-{3-méthylidène-1,7,7-triméthyl-bicyclo [2.2.1]heptan-2-one} : 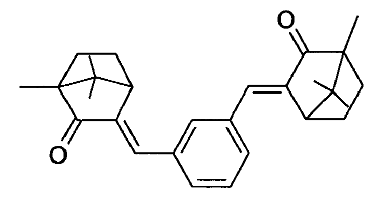 Phénylène bis méthylidène camphre sulfonamide (L'Oréal FR
2 529 887) tel le 1,4-phénylène-bis-3,3'-méthylidène camphre-10,10'-sulfonamide d'éthyle ou de 2-éthylhexyle : 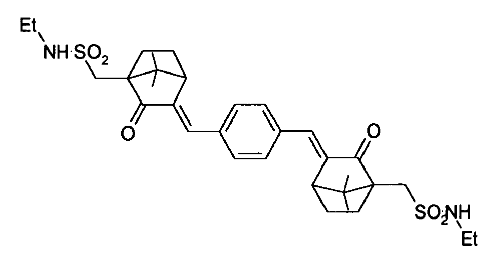 ou 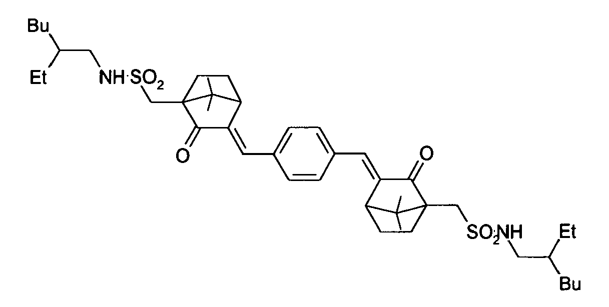 Phénylène bis méthylidène cinéole (E. Mariani et al, 16th IFSCC Congress, New York (1990)) tel la 1,4-phénylène-bis-{5-méthylidène-3,3-diméthyl-2-oxa-bicyclo[2.2.2]octan-6-one}: 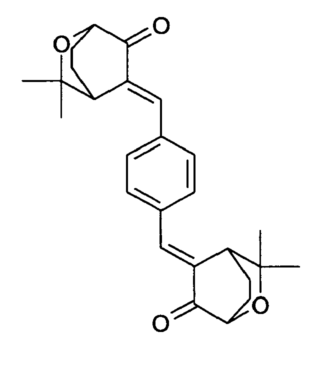 Phénylène bis méthylidène cétotricyclodécane (Merck EP 0 694 521) tel la 1,4-phénylène-bis-(octahydro-4,7-méthano-6-indèn-5-one) : 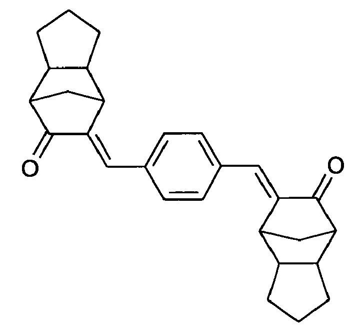 Phénylène bis alkylène cétone (Kao JP 04 134 041) telle que la 1,4-phénylène-bis-(4,4-diméthyl-pent-1-èn-3-one) : 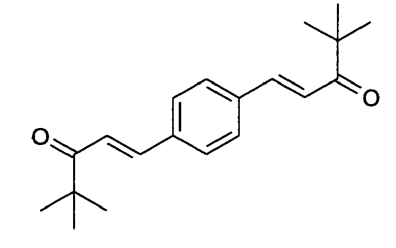 Phénylène bis méthylidène furanone (L'Oréal FR 2 638 354) telle que la 1,4-phénylène-bis-(4-méthylidène-2,2,5,5-tétraméthyl-dihydrofuran-3-one) : 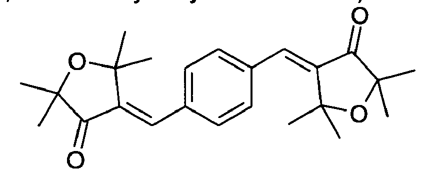 Phénylène bis méthylidène quinuclidinone (Merck EP 0 714 880) telle que la 1,4-phénylène-bis-{2-méthylidène-1-aza-bicyclo [2.2.2]octan-3-one}:

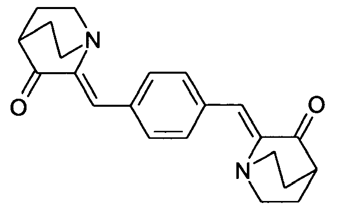

A titre de composés de formule (XII), on peut mentionner les familles suivantes :
- bis benzylidène cycloalcanone telle que la 2,5-dibenzylidène-cyclopentanone : 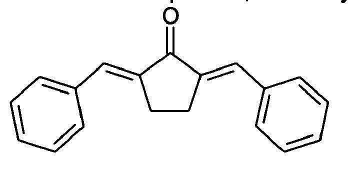 gamma pyrone (Kao JP 04 290 882) telle que la 2,6-bis-(3,4-diméthoxy-phényl)-pyran-4-one :

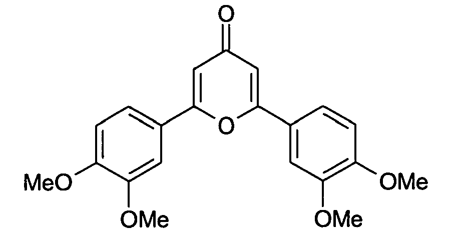

Une autre famille de filtres insolubles utilisables dans la présente invention sont les dérivés amides, sulfonamides et carbamate d'acrylonitrile correspondant à la formule suivante 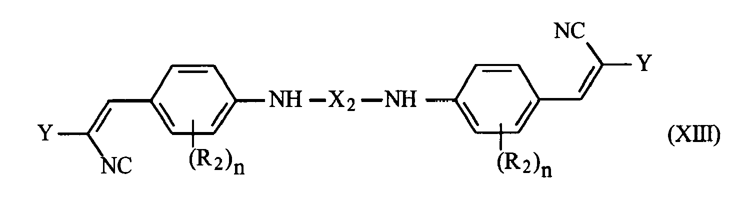 dans laquelle
X₂ représente un radical divalent de formule -(C=O)-R'₃-(C=O)- ,
-SO2-R"₃-SO₂- ou -(C=O)-O-R"₃-O-(C=O)-,
Y représente un radical -(C=O)-R₄ ou -SO₂R₅,
R₂ représente un groupe alkyle en C1-C8, linéaire ou ramifié,
n vaut 0, 1 ou 2,
R'₃ représente une simple liaison ou R"3
R"₃ représente un radical divalent alkylène en C₁-C₃₀ ou alcénylène en C₃-C₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁-C₃₀, linéaire ou ramifié, ou un noyau phényle pouvant être substitué par des radicaux alkyle ou alcoxy en C₁-C₄,
R₆ représente un radical alkyle en C₁-C₃₀ ou alcényle en C₃-C₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

Bien que dans la formule (XIII) ci-dessus, seuls soient représentés les isomères dans lesquelles le substituant cyano est en position cis par rapport au substituant para-amino-phényle, cette formule doit être comprise comme englobant également les isomères trans correspondants pour chacune des deux double liaisons et de façon indépendante, les substituants cyano et para-amino-phényle peuvent être en configuration cis ou trans l'un par rapport à l'autre.

Une autre famille de filtres organiques insolubles utilisables selon la présente invention est formée par les dérivés de phénylène-bis-benzoxazinone de formule 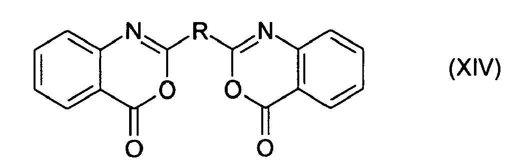 dans laquelle R représente un reste aromatique divalent choisi parmi les formules (e) à (h) suivantes : 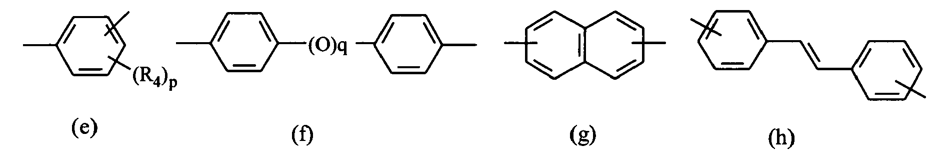 dans lesquelles :
chacun des symboles R₄ représente indépendamment un groupe OH, un atome d'halogène, un groupe alkyle en C₁-C₆, linéaire ou ramifié et contenant éventuellement un atome de silicium, un groupe alcoxy en C₁-C₆, linéaire ou ramifié et contenant éventuellement un atome de silicium, un groupe alcoxycarbonyle en C₁-C₅, linéaire ou ramifié, ou un groupe alkylsulfonamide en C₁-C₆, linéaire ou ramifié et contenant éventuellement un atome de silicium ou une fonction aminoacide,
p représente un nombre entier compris entre 0 et 4 inclus,
q représente 0 ou 1.

A titre d'exemples de composés de formule (XIV), insolubles, filtrant le rayonnement UV, on peut mentionner les dérivés suivants :
2,2'-p-phénylène bis(3,1-benzoxazin-4-one), produit commercial CYASORB® UV-3638 de la société CYTEC,
2,2'-(4,4'-biphénylène) bis (3,1-benzoxazin-4-one),
2,2'-(2,6-naphthylène) bis (3,1-benzoxazin-4-one).

Une autre famille particulière de filtres organiques insolubles sont les sels de métaux polyvalents (par exemple Ca2+, Zn2+, Mg 2+, Ba2+, A13+ ou Zr4+) de filtres organiques sulfonés ou carboxylés tels que les sels de métaux polyvalents de dérivés sulfonés de benzylidène-camphre tels que ceux décrits dans la demande FR-A 2 639 347, les sels de métaux polyvalents de dérivés sulfonés de benzimidazole tels que ceux décrits dans la demande EP-A-893 119, et les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que ceux décrits dans les demandes de brevet WO93/10753, WO93/11095 et WO95/05150.

On choisira plus particulièrement comme filtre organique insoluble les les dérivés de méthylène-bis-(hydroxyphénylbenzotriazole) de structure suivante : 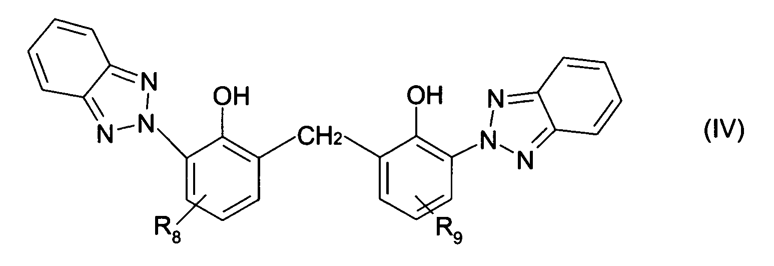 dans laquelle R⁸ et R⁹, identiques ou différents, représentent chacun un radical alkyle en C₁-C₁₈ pouvant être substitué par un ou plusieurs radicaux choisis parmi alkyle en C₁-C₄, cycloalkyle en C₅-C₁₂ ou aryle et plus particulièrement le composé (a) de nomenclature 2,2' -méthylène-bis-[6-(2H-benzotriazol-2-yl)-4-(1,1,3,3-tetraméthylbutyl)phénol] ou Methylène bis-Benzotriazolyl Tetramethylbutylphénol est vendu sous forme solide le nom MIXXIM BB/100 par le société FAIRMOUNT CHEMICAL et sous forme micronisée sous le nom TINOSORB M par la société par CIBA SPECIALTY CHEMICALS.

Les filtres organiques insolubles selon l'invention se présentent en général sous forme de particules de taille moyenne allant de 10 nm à 5 µm . Plus préférentiellement, leur taille moyenne varie de 10 nm à 2 µm, et en particulier entre 20 nm et 1,5 µm, et idéalement entre 30 nm et 1,0 µm.

D'une manière générale, la taille moyenne des particules correspondra au diamètre moyen de la distribution en nombre.

La taille moyenne des particules peut être déterminée par toute méthode classique telle que les méthodes optiques (diffusion quasi-élastique ou diffusion laser), les méthodes par centrifugation ou les méthodes de visualisation microscopique et analyse d'image.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent en particulier être obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure CₙH ₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C1-C12 d'un composé de structure CₙH₂ₙ₊₁ O(C₆H₁₀O5)ₓH et plus précisément un ester obtenu par réaction d'un acide carboxylique en C₁-C₁₂ tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre insoluble dans sa forme micronisée.

On peut également utiliser pour améliorer la dispersibilité des filtres organiques insolubles dans le support cosmétique des copolymères amphiphiles comportant au moins une séquence hydrophile et au moins une séquence hydrophobe, comme ceux décrits dans la demande de brevet EP1353642.

Le ou les filtres UV insolubles de l'invention sont présents de préférence à une concentration totale comprise entre 0,1 et 25 % en poids environ, et de préférence entre 0,2 et 20 % en poids environ, par rapport au poids total de la composition.

Les compositions conformes à l'invention peuvent comporter en plus d'autres agents photoprotecteurs organiques complémentaires actifs dans l'UVA et/ou l'UVB hydrophiles
ou lipophiles dans les solvants cosmétiques couramment utilisés.

Les agents photoprotecteurs organiques complémentaires sont notamment choisis parmi les anthranilates ; les dérivés cinnamiques ; les dérivés salicyliques, les dérivés du camphre ; les dérivés de la benzophénone; les dérivés de β,β-diphénylacrylate ; les dérivés de triazine ; les dérivés de benzotriazole ; les dérivés de benzalmalonate notamment ceux cités dans le brevet US5624663; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés salicyliaues :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre:

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,

### Dérivés de triazine :

2,4,6-tris(4'-amino benzalmalonate de dinéopentyle)-s-triazine
la 2,4,6-tris-(4'-amino benzalmalonate de düsobutyle)-s- triazine.
Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,

### Dérivés anthranili4ues :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-iminoj-6-(2-ethylhexyl)-imino-1,3,5-triazine vendu sous le nom d'Uvasorb K2A par Sigma 3V
et leurs mélanges.

Les agents photoprotecteurs organiques complémentaires préférentiels sont choisis parmi
Ethylhexyl Methoxycinnamate
Butyl Methoxydibenzoylmethane
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
Drometrizole Trisiloxane
Di-néopentyl 4'-méthoxybenzalmalonate
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-iminoj-6-(2-ethylhexyl)-imino-1,3,5-triazine
et leurs mélanges.

Les compositions conformes à l'invention peuvent comporter en plus d'autres agents photoprotecteurs inorganiques.

Les agents photoprotecteurs inorganiques sont choisis parmi les pigments (taille moyenne des particules primaires : généralement entre 5 nm et 100 nm, de préférence entre 10 nm et 50 nm) d'oxydes métalliques enrobés ou non comme par exemple des pigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. De tels pigments d'oxydes métalliques, enrobés ou non enrobés, sont en particulier décrits dans les demandes de brevets EP 518 772 et EP 518 773.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une lotion, d'un sérum, d'une pâte, de poudre, de bâtonnet solide et éventuellement être conditionnée en aérosol et se présenter sous forme de mousse ou de spray. Ces compositions sont préparées selon les méthodes usuelles.

Selon un mode particulier de réalisation de l'invention, la composition selon l'invention se présente sous forme d'une émulsion et comporte alors au moins une phase huileuse. La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant ,quand ils sont présents, le sont généralement, en une proportion allant de 0,1 à 30 % en poids, de préférence de 0,3 à 20 % en poids et mieux de 0,5 à 15 % en poids par rapport au poids total de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stéarate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, pour stabiliser lesdites émulsions par exemple des polymères amphiphiles, des charges, des épaississants ou gélifiants.

Quand la composition de l'invention est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

La phase huileuse peut aussi comporter un ou plusieurs corps gras choisi par exemple parmi les alcools gras (alcool cétylique, l'alcool stéarylique, alcool cétéarylique), les acides gras (acide stéarique) ou les cires (paraffine, cires de polyéthylène, carnauba, cire d'abeilles).

La composition de l'invention peut contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de düsopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers) et les Pemulen (Copolymère acrylate/C10-C30-alkylacrylate) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de Myrtrimonium), le bromure de dodécyl-triméthylammonium, le bromure d'hexadécyl-triméthylammonium, et leurs mélanges tel que le mélange vendu sous la dénomination Cetrimide® par la société FEF CHEMICALS.Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Comme charges qui peuvent être utilisées dans la composition de l'invention, on peut citer par exemple, les pigments ; la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres de polymethylmethacrylate telles que celles coomercialisées sous la dénomination MICROPEARL M 100 par la société Matsumoto ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les poudres de polyuréthanne telles que la poudre de copolymère hexaméthylène diisocyanate/trimethylol hexyllactone commercialisée sous la dénomination Plastic Powder D-400 par la société Toshiba Pigment (Nom CTFA : HDI / Trimethylol Hexyllactone Crosspolymer) ; et leurs mélanges. Quand elles sont présentes, ces charges peuvent être en des quantités allant de 0,001 à 20 % en poids, de préférence de 0,1 à 10 % en poids et mieux de 1 à 5 % en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

La composition selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut constituer un produit pour le soin de la peau, notamment pour le visage, le cou, le contour de l'oeil, le corps ; ou bien encore un produit de maquillage de la peau tel qu'un produit du teint (notamment fond de teint), un fard à paupières, un fard à joue, un eye-liner, un produit anticernes, un produit de maquillage du corps, un produit de protection solaire ou bien un produit de nettoyage de la peau. De manière préférentielle La composition selon l'invention sera un produit de protection solaire.

La composition est généralement non rincée, mais elle peut être rincée si elle constitue un produit de nettoyage notamment moussant.

L'invention a aussi pour objet un procédé de traitement cosmétique d'une matière kératinique telle que la peau, des cils, des sourcils, des ongles ou des muqueuses,
caractérisé en ce qu'on applique sur la matière kératinique, une composition telle que définie ci-dessus.

Selon un autre aspect, l'invention concerne également un ensemble cosmétique comprenant :
i) un récipient délimitant au moins un compartiment, ledit récipient étant fermé par un élément de fermeture ; et
ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Altemativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Les compositions selon l'invention peuvent se présenter sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

L'invention va maintenant être décrite en référence aux exemples suivants donnés à titre illustratif et non limitatif. Dans ces exemples, sauf indication contraire, les quantités sont exprimées en pourcentages pondéraux.

On a réalisé les formulations solaires suivantes ; les quantités sont indiquées en pourcentages en poids :

| **Compositions** | **Ex 1** | **Ex 2** | **Ex 3** | **Ex 4** | **Ex 5** |
|---|---|---|---|---|---|
| **PHASE A** | | | | | |
| Polydiméthylsiloxane | 0,5 | 0,5 | 0,5 | 0,5 | 0,5 |
| Conservateurs | 1,0 | 1,0 | 1,0 | 1,0 | 1,0 |
| Acide stéarique | 1,5 | 1,5 | 1,5 | 1,5 | 1,5 |
| Mélange monostéarate de glycéryle/stéarate-PEG (100 OE) | 1,0 | 1,0 | 1,0 | 1,0 | 1,0 |
| Mélange cetylstéarylglucoside/alcool cetylstéarylique | 2,0 | 2,0 | 2,0 | 2,0 | 2,0 |
| Benzoate d'alcools en C₁₂/C₁₅ | 5,0 | - | 5,0 | 5 ,0 | - |
| Isohexadécane | 1,0 | 1,0 | 1,0 | 1,0 | 1,0 |
| Alcool cétylique | 0,5 | 0,5 | 0,5 | 0,5 | 0,5 |
| Butyl Methoxydibenzoylmethane | 2,0 | 2,5 | 2,0 | 2,0 | 2,0 |
| Octocrylene | 9,0 | 10,0 | 9,0 | 10,0 | 10,0 |
| Drometrizole Trisiloxane | 1,0 | - | 4,0 | - | - |
| Methylène bis-Benzotriazolyl Tetramethylbutylphénol | 1,0 | 3,0 | 1,0 | 3,0 | 3,0 |
| TiO₂ | 3,0 | 5,0 | - | 5,0 | 5,0 |

| **PHASE B** | | | | | |
|---|---|---|---|---|---|
| N-(2-hydroxyléthyl)urée | 5,0 | 10,0 | 5,0 | 5,0 | 10,0 |
| Eau désionisée | qsp 100 | qsp 100 | qsp 100 | qsp 100 | qsp 100 |
| Sequestrant | 0,1 | 0,1 | 0,1 | 0,1 | 0,1 |
| Glycérine | 5,0 | 5,0 | 5,0 | 5,0 | 5,0 |
| Gomme de Xanthane | 0,2 | 0,2 | 0,2 | 0,2 | 0,2 |
| Phosphate de monocétyle | 1,0 | 1,0 | 1,0 | 1,0 | 1,0 |

| **PHASE C** | | | | | |
|---|---|---|---|---|---|
| Copolymère acide acrylique/méthacrylate de stéaryle | 0,2 | 0,2 | 0,2 | 0,2 | 0,2 |
| Triéthanolamine | qs | qs | qs | qs | qs |

### Mode opératoire

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotorstator (appareil de la société Moritz). On incorpore la phase C et on laisse revenir à température ambiante sous agitation modérée. On introduit la triéthanolamine de façon à ajuster le pH à la valeur désirée en fin de fabrication.

## Revendications

**1.** Composition comprenant dans un support cosmétiquement acceptable au moins un système filtrant les radiations UV, **caractérisée par le fait qu'**elle contient :
(a) au moins un filtre UV organique insoluble et
(b) au moins une hydroxyalkylurée de formule (1) 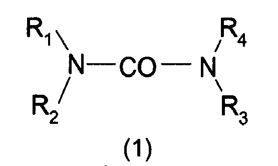 dans laquelle Rₐ, R_{b}, R_{c} et R_{d} représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux Rₐ-R_{d} représentant un groupe hydroxyalkyle, ainsi que leurs sels, solvats et isomères.

**2.** Composition selon la revendication 1, **caractérisée en ce que** Rₐ est un groupe hydroxyalkyle et R_{b}, R_{c} et R_{d} représentent, indépendamment les uns des autres, un atome d'hydrogène ou un groupe alkyle en C₁-C₄.

**3.** Composition selon la revendication 2, **caractérisée en ce que** Rₐ est un groupe hydroxyalkyle et R_{b}, R_{c}et R_{d} représentent chacun un atome d'hydrogène.

**4.** Composition selon l'une quelconque des revendications 1 à 3, **caractérisée en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée; la N-méthyl-N-(1,3,4,5,6-pentahydroxy-2-hexyl)-urée ; la N-méthyl-N'-(1-hydroxy-2-méthyl-2-propyl)- urée; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée; la N-(1,3-dihydroxy-2-propyl)-N'-(2-hydroxyéthyl)- urée; la N,N-Bis-(2-hydroxyéthyl)-N',N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

**5.** Composition selon l'une quelconque des revendications 1 à 4, **caractérisée en ce que** l'hydroxyalkyl urée est la N-(2-hydroxyéthyl)-urée.

**6.** Composition selon l'une quelconque des revendications 1 à 5 où le ou les filtres hydrophiles sont présents à des teneurs allant de 0,01 à 50% en poids et plus préférentiellement de 0,1 à 20% en poids et encore plus préférentiellement de 0,1 à 10% en poids par rapport au poids total de la composition.

**7.** Composition selon l'une quelconque des revendications 1 à 6, où le ou les filtres UV organiques insolubles sont choisis parmi ceux du type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

**8.** Composition selon l'une quelconque des revendications 1 à 6, où le ou les filtres UV organiques insolubles sont choisis parmi ceux les sels de métaux polyvalents de filtres organiques sulfonés ou carboxylés.

**9.** Composition selon la revendication 8, où le filtres UV insolubles sont choisis parmi les sels de métaux polyvalents de dérivés sulfonés de benzylidène-camphre ; les sels de métaux polyvalents de dérivés sulfonés de benzimidazole et les sels de métaux polyvalents de dérivés d'acide cinnamique .

**10.** Composition selon l'une quelconque des revendications 1 à 6, où le ou les filtres UV organiques insolubles sont choisis les dérivés de méthylène-bis-(hydroxyphénylbenzotriazole) de structure suivante : 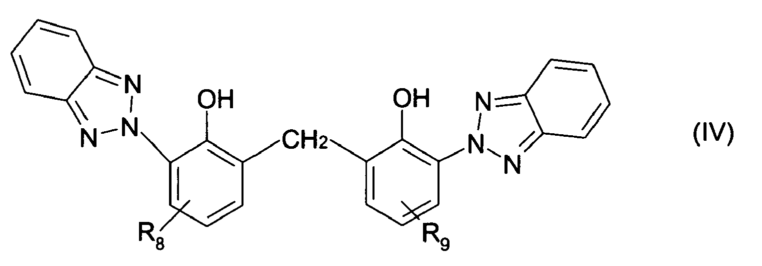 dans laquelle R⁸ et R⁹, identiques ou différents, représentent chacun un radical alkyle en C₁-C₁₈ pouvant être substitué par un ou plusieurs radicaux choisis parmi alkyle en C₁-C₄, cycloalkyle en C₅-C₁₂ ou aryle.

**11.** Composition selon la revendication 10, où le filtre UV organique insoluble est le 2,2'-méthylène-bis-[6-(2H-benzotriazol-2-yl)-4-(1,1,3,3-tetraméthylbutyl)phénol].

**12.** Composition selon l'une quelconque des revendications 1 à 6, où le ou les filtres UV organiques insolubles sont sous forme de particules de taille moyenne allant de 10 nm à 5 µm.

**13.** Composition selon la revendication 12, où la taille moyenne varie de 10 nm à 2 µm, et en particulier de 20 nm à 1,5 µm, et plus particulièrement de 30 nm à1,0 µm.

**14.** Composition selon l'une quelconque des revendications 1 à 13, où le ou les filtres UV organiques insolubles sont susceptibles d'être obtenus par un procédé de broyage d'un filtre UV organique insoluble sous forme de particules de taille grossière en présence d'un tensio-actif.

**15.** Composition selon la revendication 14, où le tensio-actif est choisi parmi les alkylpolyglucosides de structure CₙH ₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6.

**16.** Composition selon la revendication 15, où le tensio-actif est utilisé à une concentration de allant de 1 à 50% en poids et plus préférentiellement de 5 à 40% en poids par rapport au filtre insoluble.

**17.** Composition selon l'une quelconque des revendications 1 à 16, contenant en plus au moins un copolymère amphiphile comportant au moins une séquence hydrophile et au moins une séquence hydrophobe.

**18.** Composition selon l'une quelconque des revendications 1 à 17, où le ou les filtres UV insolubles sont présents à une concentration totale comprise entre 0,1 et 25 % en poids environ, et de préférence entre 0,2 et 20 % en poids environ, par rapport au poids total de la composition.

**19.** Composition selon l'une quelconque des revendications précédentes, **caractérisée en ce qu'**elle constitue un produit pour le soin de la peau, un produit de maquillage de la peau, un produit de protection solaire, un produit de nettoyage de la peau.

**20.** Composition selon la revendication 19, **caractérisée en ce qu'**elle constitue un produit de protection solaire.

**21.** Procédé de traitement cosmétique d'une matière kératinique, **caractérisé par le fait qu'**on applique sur la matière kératinique, une composition cosmétique selon l'une quelconque des revendications 1 à 20.

**22.** Ensemble cosmétique comprenant
(i) un récipient délimitant au moins un compartiment, ledit récipient étant fermé par un élément de fermeture ; et
(ii) une composition selon l'une quelconque des revendications 1 à 20 et disposée à l'intérieur dudit compartiment.

**23.** Utilisation d'au moins une hydroxyalkylurée de formule (1) telle que définie dans les revendications précédentes dans une composition comprenant dans un support cosmétiquement acceptable au moins un filtre UV organique insoluble, dans le but d'améliorer le confort après application en particulier la douceur au toucher.
